# FASCICULE DE BREVET EUROPEEN

(11) **EP 3 716 917 B1**
(45) Date de publication et mention de la délivrance du brevet: **03.01.2024**
(21) Numéro de dépôt: 18833259.7
(22) Date de dépôt: 27.11.2018
(51) Int. Cl.: A61F 2/30, A61F 2/40

(54) **IMPLANT GLÉNOÏDIEN POUR PROTHÈSE D'ÉPAULE INVERSÉE**
GLENOIDIMPLANTAT FÜR EINE INVERTIERTE SCHULTERPROTHESE
GLENOID IMPLANT FOR INVERTED SHOULDER PROSTHESIS

(30) Priorité: 28.11.2017 FR 1761301
(43) Date de publication de la demande: 07.10.2020
(73) Titulaire: Shoulder Friends Institute, 75008 Paris (FR)
(72) Inventeur: LEFEBVRE, Yves, 67000 Strasbourg (FR); AUDEBERT, Stéphane, 59268 Blecourt (FR); BARTH, Johannes, 38240 Meylan (FR); CHAROUSSET, Christophe, 75017 Paris (FR); GARRET, Jérôme, 69760 Limonest (FR); GODENECHE, Arnaud, 69450 Saint Cyr Au Mont D'Or (FR); GUERY, Jacques, 58000 Nevers (FR); JOUDET, Thierry, 33500 Libourne (FR); GALLINET, David, 25870 Geneuille (FR)
(74) Mandataire: Germain Maureau
(86) Numéro de dépôt international: PCT/FR2018/052996
(87) Numéro de publication internationale: WO 2019/106279

(56) Documents cités:
- EP-A1- 1 332 734
- EP-A1- 2 601 912
- WO-A1-2015/103090
- US-A1- 2004 220 673

## Description

La présente invention est définie dans la revendication 1 et se rapporte à un implant glénoïdien pour prothèse d'épaule inversée.

Classiquement, un tel implant glénoïdien comprend une embase prévue pour être ancrée sur une glène d'une omoplate, et une glénosphère montée sur l'embase et définissant une surface d'articulation convexe en portion sphérique.

Il est connu, notamment du document EP 2 601 912 de prévoir un montage de la glénosphère sur l'embase par un coincement mutuel entre des portées coniques femelle et mâle, et de prévoir une vis d'ancrage principale traversant l'embase et conformée pour l'ancrage de l'embase sur la glène. L'implant glénoïdien de ce document EP 2 601 912 présente cependant l'inconvénient qu'aucun verrouillage de la glénosphère sur l'embase n'est prévu.

Pour résoudre en partie cet inconvénient, la demande internationale WO 2015/103090 propose, en plus d'un coincement mutuel entre des portées coniques, d'employer une vis de verrouillage traversant la glénosphère pour verrouiller cette dernière sur l'embase. Dans ce document, une vis d'ancrage principale est engagée par-dessous (du côté de la face interne de l'embase prévue pour être en contact avec la surface glénoïdienne native préparée) pour être bloquée sur l'embase par un circlips ou par un filetage proximal ayant un pas à gauche tandis que le filetage distal servant à l'ancrage dans la glène présente un pas à droite, et en outre la vis de verrouillage est vissée dans l'embase.

Cependant, l'implant glénoïdien de ce document WO 2015/103090 présente un premier inconvénient qui est l'absence de guidage de la glénosphère lors de son positionnement sur l'embase, et un second inconvénient qui est que les efforts de compression dans la vis d'ancrage principale passent par le circlips ou par le filetage proximal, nuisant ainsi à la tenue mécanique globale de l'implant glénoïdien.

L'état de la technique peut également être illustré par l'enseignement du document EP 1 332 734 qui divulgue un implant glénoïdien comprenant une embase munie d'un plot d'ancrage fixe pour un ancrage dans la glène, et une glénosphère montée sur l'embase par un coincement mutuel entre des portées coniques femelle et mâle. Il est également prévu une vis de verrouillage pour verrouiller la glénosphère sur l'embase dans une position verrouillée. La vis de verrouillage est tout d'abord accrochée par le dessous sur la glénosphère au moyen d'un filetage supérieur vissé dans un orifice taraudé de la glénosphère et d'un filetage intermédiaire vissé dans une douille qui est elle-même vissée sur le dessous de la glénosphère. De par ces trois vissages simultanés, il est bien évident qu'une telle solution est problématique à réaliser, car il nécessite une très grande précision pour que tous les vissages coïncident simultanément. Ensuite, la vis de verrouillage est vissé jusqu'à ce que le filetage supérieur quitte l'orifice taraudé dans la glénosphère, et que le filetage intermédiaire quitte la douille pour venir se visser dans un taraudage prévu dans le plot d'ancrage fixe. Un tel implant glénoïdien présente l'inconvénient que l'ancrage ne se fait que par un plot d'ancrage fixe, donc sans vissage, et aussi les inconvénients de la complexité et du surcoût liés à l'emploi d'une pièce intermédiaire qui est la douille.

Il est aussi connu du document US 2004/0220673 d'employer un implant glénoïdien comprenant une embase ancrée dans la glène au moyen d'une vis d'ancrage et une glénosphère montée sur l'embase par un coincement mutuel entre des portées coniques femelle et mâle et verrouillée sur l'embase au moyen d'une vis de verrouillage qui se visse dans la vis d'ancrage. Lors du montage de la glénosphère sur l'embase, cette glénosphère est indexée au moyen de la tête de la vis d'ancrage qui est conique et qui coopère avec un trou de centrage prévu au centre de la glénosphère. Un inconvénient de cet implant glénoïdien est que la tête de la vis d'ancrage doit saillir sur le dessus de l'embase suffisamment pour pouvoir guider la glénosphère, et cette saillie peut être gênante pour le chirurgien lors d'opérations annexes à effectuer en amont de la pose de la glénosphère sur l'embase, comme par exemple lors du vissage de vis périphériques d'ancrage dans la glène.

La présente invention a pour but de résoudre en tout ou partie les inconvénients précités en proposant un implant glénoïdien qui permette un guidage et un verrouillage de la glénosphère sur l'embase, tout en offrant une tenue mécanique améliorée.

Un autre but de l'invention est de proposer un implant glénoïdien conformé pour ne pas gêner des opérations annexes à effectuer en amont du verrouillage de la glénosphère sur l'embase.

A cet effet, elle propose un implant glénoïdien pour prothèse d'épaule inversée, comprenant :
- une embase prévue pour être ancrée sur une glène d'une omoplate et présentant un orifice principal traversant l'embase ;
- une glénosphère définissant une surface d'articulation convexe en portion sphérique et présentant un orifice central traversant la glénosphère, où ladite glénosphère est montée sur ladite embase par un coincement mutuel entre des portées coniques femelle et mâle prévues sur la glénosphère et sur l'embase ;
- une vis d'ancrage principale traversant l'orifice principal de ladite embase et conformée pour l'ancrage de l'embase sur la glène, où ladite vis d'ancrage principale comprend une tête principale et une tige d'ancrage présentant un segment d'extrémité muni d'un filetage extérieur ;
- une vis de verrouillage, coaxiale à la vis d'ancrage principale, traversant l'orifice central de la glénosphère pour verrouiller la glénosphère sur l'embase dans une position verrouillée, où ladite vis de verrouillage comprend une tête proximale et une tige de verrouillage présentant un segment intermédiaire muni d'un filetage extérieur et prolongé par un segment d'extrémité ;
dans lequel l'implant glénoïdien selon l'invention est remarquable en ce que :
- la vis d'ancrage principale présente un trou interne débouchant dans la tête principale et présentant successivement, en partant de sa tête principale et en direction du segment d'extrémité de sa tige d'ancrage, une portion d'entrée conformée pour coopérer avec un outil de vissage, une portion intermédiaire munie d'un taraudage et une portion terminale ;
- la tige de verrouillage a son segment d'extrémité qui présente une longueur supérieure à une longueur cumulée de la portion d'entrée et de la portion intermédiaire du trou interne de la vis d'ancrage principale, de sorte que ledit segment d'extrémité de la tige de verrouillage est prévu pour glisser à l'intérieur de la portion terminale du trou interne de la vis d'ancrage principale pour centrer les portées coniques entre elles, et que le segment intermédiaire de la tige de verrouillage est prévu pour ensuite coopérer par vissage avec la portion intermédiaire du trou interne de la vis d'ancrage principale pour verrouiller le coincement mutuel des portées coniques, la tête proximale de la vis de verrouillage étant en butée sur une portée annulaire prévue à l'intérieur de l'orifice central de la glénosphère ;
- l'orifice central de la glénosphère présente successivement, en partant de la surface d'articulation convexe en direction de l'embase, une portion proximale conformée pour recevoir intégralement la tête proximale de la vis de verrouillage, et une portion distale munie d'un taraudage complémentaire du filetage extérieur du segment intermédiaire de la tige de verrouillage de la vis de verrouillage, où la portée annulaire de l'orifice central de la glénosphère est prévue à l'interface entre ladite portion proximale et ladite portion distale.

Ainsi, la vis de verrouillage va garantir à la fois le guidage de la glénosphère sur l'embase afin de guider les deux portées coniques en contact mutuel et centré, et le verrouillage de la glénosphère sur l'embase avec une transmission des efforts de compression qui passent par la vis d'ancrage central et également par la vis de verrouillage et par la portée annulaire prévue à l'intérieur de l'orifice central de la glénosphère, garantissant une tenue mécanique accrue comparativement à l'art antérieur.

Grâce à l'invention, le guidage de la glénosphère va permettre de faciliter le travail du chirurgien, qui travaille parfois en appui sur des écarteurs selon l'accès à l'articulation retenu qui dépend de la voie d'abord chirurgicale.

Dans l'ensemble de la description, les longueurs sont mesurées le long des axes longitudinaux et coaxiaux de la vis d'ancrage principale et de la vis de verrouillage, en situation et en position verrouillée.

Ainsi, la vis de verrouillage peut être préalablement positionnée sur la glénosphère, en faisant passer par vissage le segment intermédiaire de la tige de verrouillage de la vis de verrouillage à travers la portion distale taraudée de l'orifice central de la glénosphère, bloquant ainsi la vis de verrouillage en coulissement vers le haut (blocage avec la portion distale taraudée) et vers le bas (blocage avec la portée annulaire).

Avantageusement, la portion distale de l'orifice central de la glénosphère présente une longueur inférieure à celle du segment intermédiaire de la tige de verrouillage de la vis de verrouillage.

Selon une autre caractéristique, la tige de verrouillage de la vis de verrouillage présente un segment proximal s'étendant entre la tête proximale et le segment intermédiaire.

Ce segment intermédiaire contribue à autoriser un degré de coulissement à la vis de verrouillage pour faciliter le montage et pour permettre l'impaction de la glénosphère nécessaire au montage par coincement mutuel entre les portées coniques femelle et mâle.

Avantageusement, la portion distale taraudée de l'orifice central de la glénosphère présente une longueur inférieure à celle du segment proximale de la tige de verrouillage de la vis de verrouillage.

Dans une réalisation particulière, la vis d'ancrage principale et la vis de verrouillage sont coaxiales aux portées coniques mâle et femelle.

Dans un mode de réalisation particulier, en position verrouillée, la tête principale de la vis d'ancrage principale vient en butée sur une portée annulaire prévue à l'intérieur de l'orifice principal de l'embase, favorisant une transmission des efforts de compression dans la vis d'ancrage principale via cette portée annulaire.

Selon une possibilité de l'invention, la tête principale de la vis d'ancrage principale présente un filetage extérieur, et l'orifice principal de l'embase présente une portion d'entrée débouchant sur une face externe de l'embase faisant face à la glénosphère, où ladite portion d'entrée est munie d'un taraudage complémentaire dudit filetage extérieur de ladite tête principale, de sorte que ladite tête principale est vissée dans ladite portion d'entrée.

Selon une autre possibilité de l'invention, le filetage extérieur de la tête principale de la vis d'ancrage principale présente un pas de filetage inférieur à celui du filetage extérieur du segment d'extrémité de la tige d'ancrage, ce qui permet d'optimiser l'ancrage par vissage dans la glène du filetage extérieur du segment d'extrémité de la tige d'ancrage.

Ainsi, le filetage extérieur du segment d'extrémité de la tige d'ancrage présente un grand pas de filetage adapté à l'accroche dans de l'os spongieux, tandis que le filetage extérieur de la tête principale de la vis d'ancrage principale présente un petit pas de filetage (tel qu'un pas métrique), de sorte que chaque tour de vis ne correspond pas à la même longueur de vissage pour ces deux filetages.

Conformément à une autre caractéristique avantageuse de l'invention, l'embase présente un plot central saillant d'une face interne opposée à la glénosphère, où ledit plot central est creux et est traversé par l'orifice principal et par la vis d'ancrage principale dont le segment d'extrémité de la tige d'ancrage dépasse dudit plot central ; un tel plot creux étant avantageux pour favoriser l'ancrage dans la glène et permettre le centrage de la vis d'ancrage principale pour garantir une coaxialité avec la vis de verrouillage.

Avantageusement, l'orifice principal de l'embase présente une portion de sortie qui prolonge la portion d'entrée et qui traverse le plot central, et la tige d'ancrage présente un segment intermédiaire conformé pour être monté glissant et ajusté à l'intérieur de la portion de sortie de l'orifice principal de l'embase, garantissant le centrage de la vis d'ancrage principale et incidemment une coaxialité avec la vis de verrouillage.

La présente invention concerne également la caractéristique selon laquelle l'implant glénoïdien comprend en outre plusieurs vis d'ancrage secondaires conformées pour l'ancrage de l'embase sur la glène, où lesdites vis d'ancrage secondaires traversent l'embase et sont disposées en périphérie autour de la vis d'ancrage principale.

De telles vis d'ancrage périphériques servent à compléter et renforcer l'ancrage dans la glène.

Selon une possibilité, la tête principale de la vis d'ancrage principale ne dépasse pas d'une face externe faisant face à la glénosphère.

Ainsi, la vis d'ancrage principale ne déborde pas extérieurement de l'embase et ne va donc pas gêner les opérations annexes à effectuer en amont du verrouillage de la glénosphère sur l'embase, comme par exemple les opérations de vissage des vis d'ancrage secondaires décrites ci-dessus.

L'invention se rapporte également à une prothèse d'épaule inversée comprenant un implant glénoïdien conforme à l'invention, et un implant huméral prévu pour être ancré sur un humérus et comprenant une calotte hémisphérique définissant une surface d'articulation concave conformée pour une articulation avec la surface d'articulation convexe de la glénosphère de l'implant glénoïdien.

À des fins explicatives uniquement, mais ne faisant pas partie de l'invention, un procédé d'utilisation d'un implant glénoïdien pourrait comprendre les étapes suivantes;
- engager la vis d'ancrage principale dans l'orifice principal de l'embase ;
- engager la vis de verrouillage dans l'orifice central de la glénosphère jusqu'à ce que le segment d'extrémité de la tige de verrouillage dépasse de la glénosphère ;
- approcher la glénosphère de l'embase de sorte que le segment d'extrémité de la tige de verrouillage glisse à l'intérieur de la portion terminale du trou interne de la vis d'ancrage principale et que les portées coniques viennent au contact l'une de l'autre ;
- enfoncer et visser la tige de verrouillage pour que le segment intermédiaire de la tige de verrouillage coopère par vissage avec la portion intermédiaire du trou interne de la vis d'ancrage principale pour verrouiller le coincement mutuel des portées coniques, la tête proximale de la vis de verrouillage étant en butée sur la portée annulaire prévue à l'intérieur de l'orifice central de la glénosphère.

D'autres caractéristiques et avantages de la présente invention apparaîtront à la lecture de la description détaillée ci-après, d'un exemple de mise en oeuvre non limitatif, faite en référence aux figures annexées dans lesquelles :
- la figure 1 est une vue schématique en perspective et en éclatée (ou avant montage) d'un implant glénoïdien selon l'invention ;
- les figures 2 à 4 sont des vues schématiques respectivement de côté, en coupe axiale et de dessous de la glénosphère de l'implant glénoïdien de la figure 1 ;
- la figure 5 est une vue schématique en coupe axiale de la glénosphère avec la vis de verrouillage vissée en position d'attente pour l'implant glénoïdien de la figure 1 ;
- les figures 6 et 7 sont des vues schématiques respectivement en coupe axiale et de dessous d'une variante de la glénosphère avec un désaxage de son orifice central vis-à-vis de l'axe de révolution de la surface d'articulation convexe en portion sphérique ;

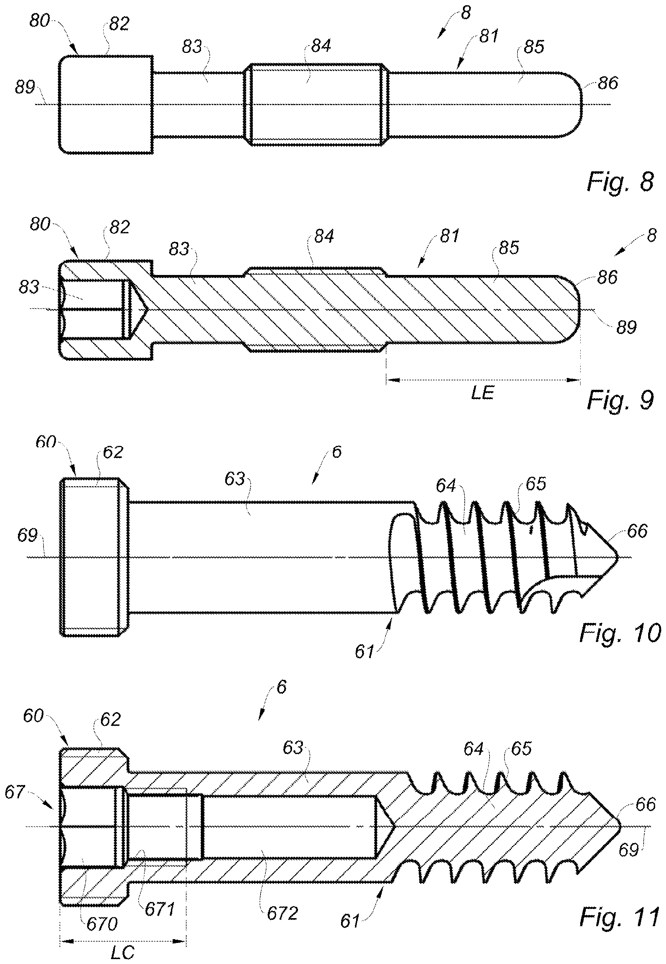

- les figures 8 et 9 sont des vues schématiques respectivement de côté et en coupe axiale de la vis de verrouillage de l'implant glénoïdien de la figure 1 ;
- les figures 10 et 11 sont des vues schématiques respectivement de côté et en coupe axiale de la vis d'ancrage principale de l'implant glénoïdien de la figure 1 ;

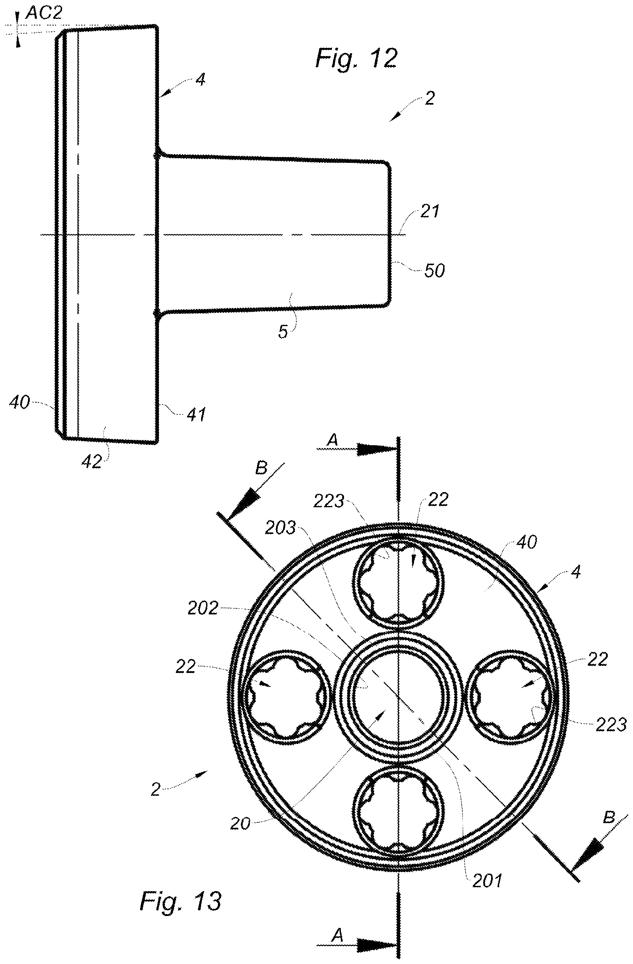

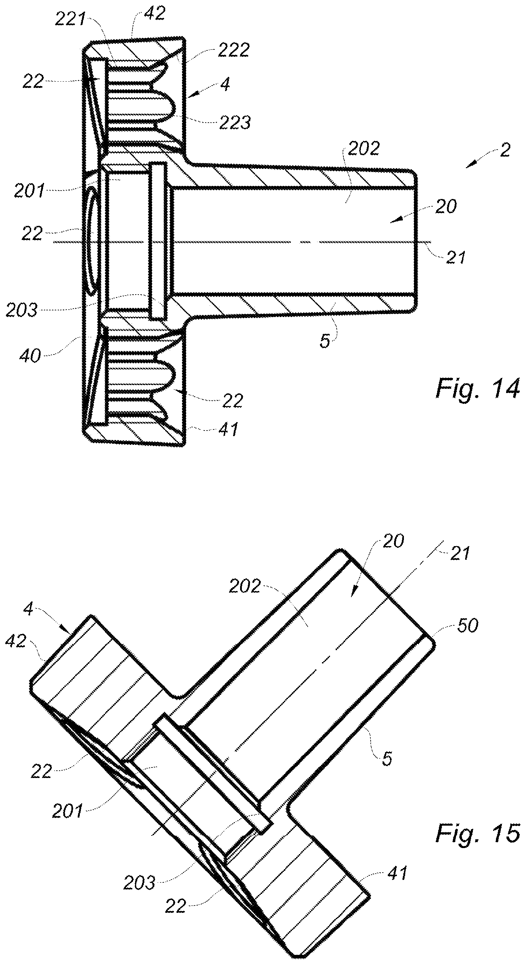

- les figures 12 à 15 sont des vues schématiques de l'embase de l'implant glénoïdien de la figure 1, respectivement de côté, de dessus, en coupe axiale selon le plan de coupe A-A de la figure 13 et en coupe axiale selon le plan de coupe B-B de la figure 13 ;
- les figures 16 à 20 sont des vues schématiques en perspective de l'implant glénoïdien de la figure 1 à différentes étapes de montage ;
- les figures 21 à 23 sont des vues schématiques en coupe axiale de l'implant glénoïdien de la figure 1 à différentes étapes de montage.

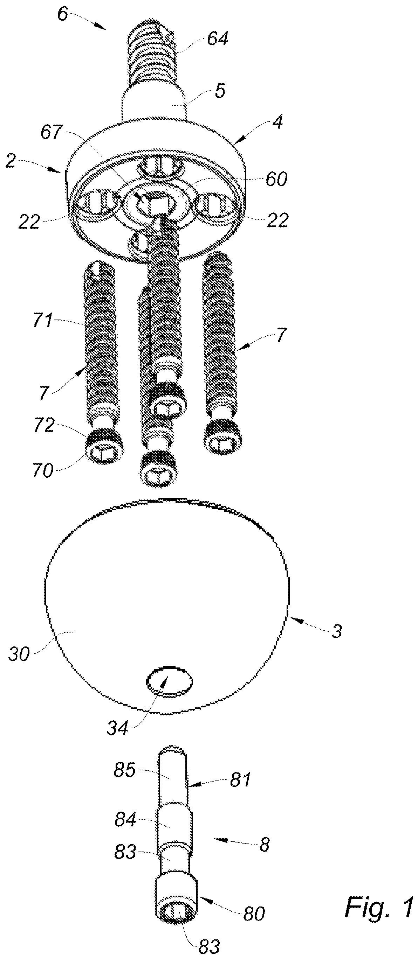

En référence à la figure 1, un implant glénoïdien 1 conforme à l'invention, prévu pour une prothèse d'épaule inversée, comprend une embase 2 et une glénosphère 3.

L'embase 2 est prévue pour être ancrée sur une glène d'une omoplate. Cette embase 2 est une pièce monobloc en matière métallique. L'embase 2 comprend une platine 4 et un plot central 5.

La platine 4 présente :
- une face externe 40 faisant face à la glénosphère 3,
- une face interne 41 opposée à la glénosphère 3 et prévue pour venir en appui sur la glène, et en particulier sur la surface réséquée de la glène ; et
- une face périphérique 42 conique formant une portée conique mâle pour un assemblage avec la glénosphère 3.

La face périphérique 42 présente un angle de conicité AC2 compris entre 4 et 7 degrés, et notamment entre 5 et 6 degrés, et forme un cône morse d'assemblage.

Le plot central 5 est un plot d'ancrage prévu pour être ancré dans la glène, et ce plot central 5 fait saillie de la face interne 41 de la platine 4. Ce plot central 5 est creux et se présente ainsi sous la forme d'un manchon tubulaire, et il présente une surface périphérique conique. Le plot central 5 présente une terminaison 50 libre.

L'embase 2 présente un orifice principal 20 traversant l'embase 2, où cet orifice principal 20 débouche dans la face externe 40 de la platine 4 et traverse la platine 4 dans son épaisseur et traverse également le plot central 5 pour déboucher dans sa terminaison 50. L'orifice central 20 est centré sur un axe central 21. Le plot central 5 est également centré sur ce même axe central 21, et présente notamment une symétrie de révolution centré sur ce même axe central 21.

L'orifice principal 20 présente successivement, en partant de la face externe 40 et en direction de la terminaison 50 :
- une portion d'entrée 201 munie d'un taraudage et débouchant sur la face externe 40 ;
- une portion de sortie 202 cylindrique et non taraudée (par exemple lisse) et débouchant sur la terminaison 50, où cette portion de sortie 202 s'étend sur au moins toute la longueur du plot central 5.

La portion de sortie 202 est de diamètre réduit comparativement à la portion d'entrée 201, de sorte que l'orifice principal 20 présente une portée annulaire 203 entre la fin du taraudage de la portion d'entrée 201 et le début de la portion de sortie 202.

L'implant glénoïdien 1 comprend en outre une vis d'ancrage principale 6 prévue pour traverser l'orifice principal 20 de l'embase 2, et même pour être fixée à l'intérieur de cet orifice principal 20 et pour déboucher de l'embase 2 afin d'assurer un ancrage principal de l'embase 2 et donc de l'implant glénoïdien 1 dans la glène.

Cette vis d'ancrage principale 6 comprend une tête principale 60 et une tige d'ancrage 61 centrées sur un axe longitudinal 69.

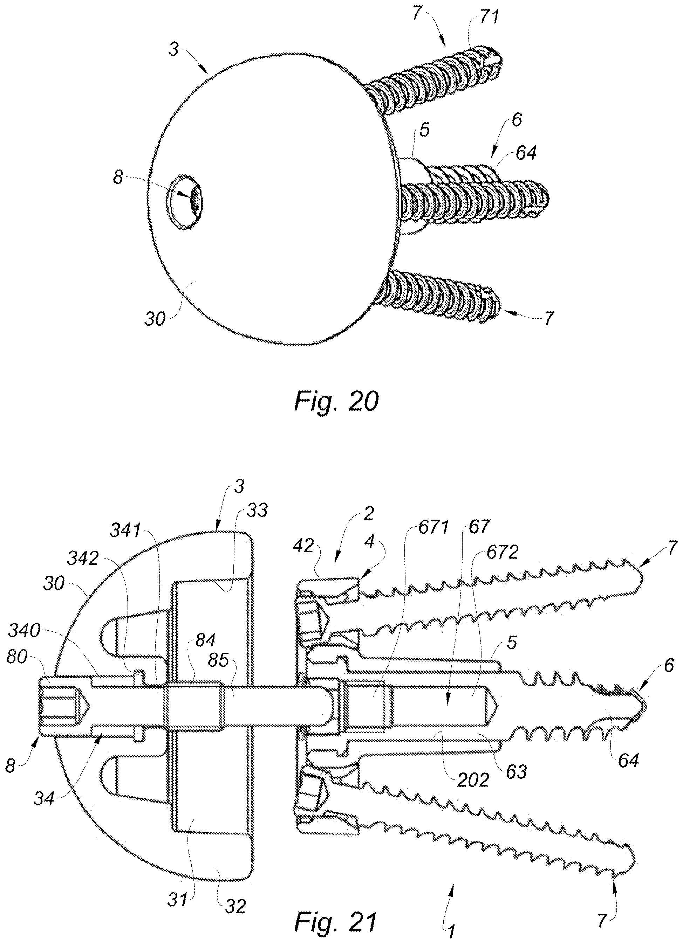

La tête principale 60 est une tête élargie qui présente un filetage extérieur 62 qui est complémentaire du taraudage de la portion d'entrée 201 de l'orifice principal 20, de sorte que cette tête principale 60 peut être vissée dans cette portion d'entrée 201 jusqu'à venir en butée sur la portée annulaire 203 dans une position verrouillée visible sur les figures 21 à 23.

Par ailleurs, et comme visible sur les figures 21 à 23 et 17 et 18, une fois que la vis d'ancrage principale 6 est en place, la tête principale 60 ne dépasse pas de la face externe 40 de la platine 4 de l'embase 2. La tête principale 60 vient même en arasement avec cette face externe 40 de la platine 4. Ainsi, la vis d'ancrage principale 6 ne gêne pas les opérations de vissage des vis d'ancrage secondaires 7 décrites ultérieurement.

La tige d'ancrage 61 présente successivement, en partant de la tête principale 60 :
- un segment intermédiaire 63 cylindrique et non fileté (par exemple extérieurement lisse) ; et
- un segment d'extrémité 64 muni d'un filetage extérieur 65 et qui se termine par une extrémité libre 66 en pointe.

Le segment intermédiaire 63 est prévu pour être monté glissant et ajusté à l'intérieur de la portion de sortie 202 de l'orifice principal 20 de l'embase 2. Ainsi, le segment intermédiaire 63 présente un diamètre externe sensiblement équivalent au diamètre interne de la portion de sortie 202, au jeu de montage près.

Le segment d'extrémité 64 est quant à lui prévu pour dépasser de la terminaison 50 du plot central 5 afin de s'ancrer dans la glène.

Le filetage extérieur 62 de la tête principale 60 présente un pas de filetage inférieur à celui du filetage extérieur 65 du segment d'extrémité 64 de la tige d'ancrage 61. Le filetage extérieur 62 est adapté à l'accroche dans de l'os spongieux, et peut présenter un pas de filetage de l'ordre de 2 millimètres, tandis que le filetage extérieur 62 de la tête principale 60 présente un pas de filetage métrique, comme par un filetage M10 à pas de 1,5 millimètres.

La vis d'ancrage principale 6 présente également un trou interne 67 borgne débouchant dans la tête principale 60 et s'arrêtant sensiblement au niveau de la fin du segment intermédiaire 63.

Ce trou interne 67 est centré sur l'axe longitudinal 69 et il présente successivement, en partant de la tête principale 60 et en direction du segment d'extrémité 64 de la tige d'ancrage 61 :
- une portion d'entrée 670 conformée pour coopérer avec un outil de vissage, une telle portion d'entrée 670 pouvant par exemple être de section polygonale et notamment de section hexagonale pour coopérer avec une clé à six pans ;
- une portion intermédiaire 671 munie d'un taraudage ; et
- une portion terminale 672 cylindrique et non taraudée (par exemple lisse).

L'embase 2 présente des orifices secondaires 22 disposés en périphérie, autour de l'orifice principal 20, où ces orifices secondaires 22 traversent la platine 4 dans son épaisseur en débouchant dans la face externe 40 et dans la face interne 41 en périphérie du plot central 5. Dans l'exemple illustré, les orifices secondaires 22 sont au nom de quatre et sont répartis angulairement tous les 90 degrés.

Chaque orifice secondaire 22 présente successivement, en partant de la face externe 40 et en direction de la face interne 41 :
- une portion d'entrée 221 munie d'un taraudage et débouchant sur la face externe 40, où des rainures axiales 223 sont ménagées dans la portion d'entrée 221 en formant des interruptions du taraudage ; et
- une portion de sortie 222 évasée et de forme conique.

L'implant glénoïdien 1 comprend en outre des vis d'ancrage secondaires 7 prévues pour traverser les orifices secondaires 22 de l'embase 2, et même pour être fixées à l'intérieur de ces orifices secondaires 22 et pour déboucher de la platine 4 afin d'assurer un ancrage secondaire de l'embase 2 et donc de l'implant glénoïdien 1 dans la glène.

Chaque vis d'ancrage secondaire 7 comprend une tête secondaire 70 et une tige d'ancrage 71 qui dépasse de la platine 4 afin de s'ancrer dans la glène.

La tête secondaire 70 est une tête élargie qui présente un filetage extérieur 72 qui est complémentaire du taraudage de la portion d'entrée 221 de l'orifice secondaire 22, de sorte que cette tête secondaire 70 peut être vissée dans cette portion d'entrée 221. La tête secondaire 70 présente une forme en coupole ce qui permet, en complément des rainures axiales 223, de régler l'inclinaison de la tige d'ancrage 71.

La glénosphère 3 définit une surface d'articulation convexe 30 en portion sphérique qui est prévue pour s'articuler sur une surface d'articulation concave d'une calotte hémisphérique d'un implant huméral prévu pour être ancré sur un humérus.

Cette glénosphère 3 présente une cavité principale 31 ouverte sur le dessous en face de l'embase 2, où cette cavité principale 31 est délimitée par une face interne périphérique 33 d'une paroi périphérique 32, où cette face interne périphérique 33 forme une portée conique femelle pour un assemblage avec l'embase 2.

Plus précisément, la glénosphère 3 est montée sur l'embase 2 selon un montage en cône morse, par un coincement mutuel entre :
- la face périphérique 42 de la platine 4 de l'embase 2 formant une portée conique mâle ; et
- la face interne périphérique 33 de la glénosphère 3 formant une portée conique femelle.

La face interne périphérique 33 présente ainsi un angle de conicité AC3 équivalent à l'angle de conicité AC2 de la face périphérique 42.

La glénosphère 3 présente en outre un orifice central 34 traversant la glénosphère 3 et débouchant, d'un côté, dans la surface d'articulation convexe 30 et, de l'autre côté, dans la cavité principale 31.

Cet orifice central 34 est centré sur un axe central 35 qui est coaxial à la face interne périphérique 33 conique. Autrement dit, la face interne périphérique 33 conique est centrée sur un axe de révolution qui est confondu ou coaxial avec cet axe central 35.

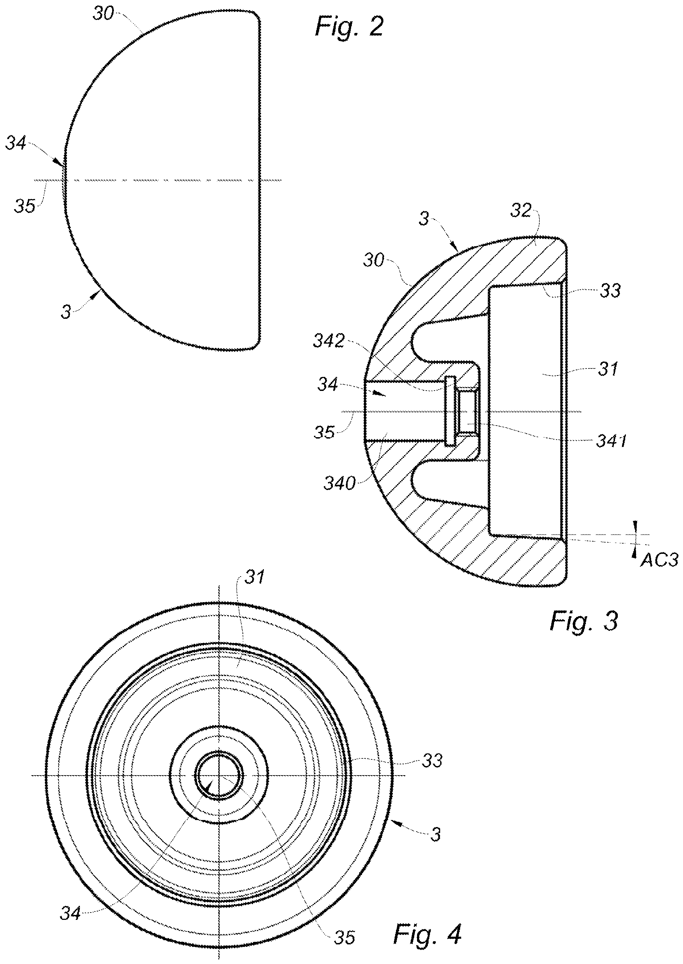

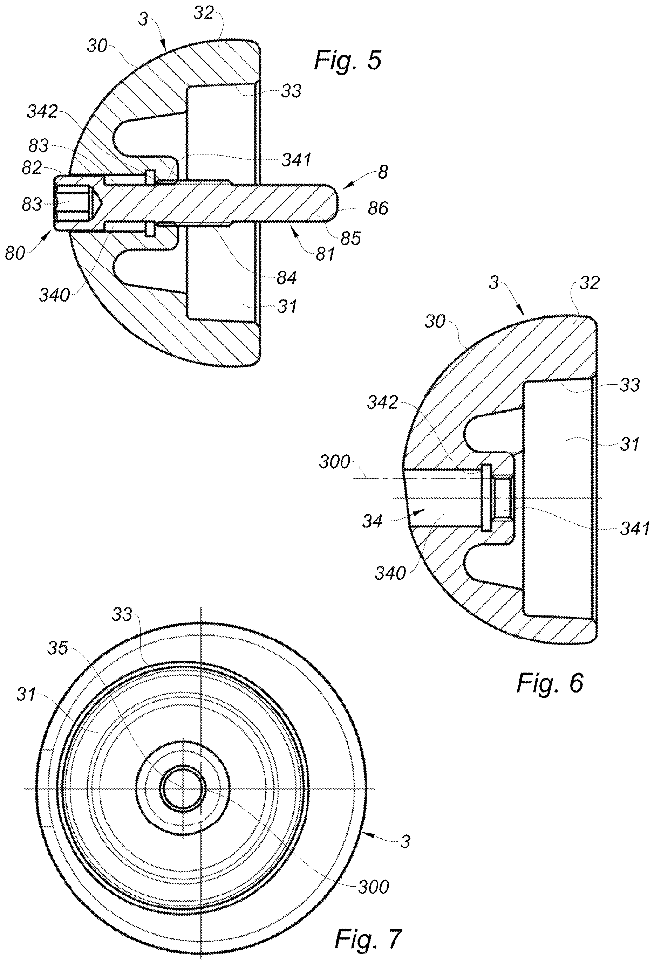

Dans l'exemple des figures 2 à 5, cet axe central 35 est également confondu avec l'axe de révolution de la surface d'articulation convexe 30, tandis que dans l'exemple des figures 6 et 7 cet axe central 35 est désaxé et parallèle par rapport à l'axe de révolution 300 de la surface d'articulation convexe 30.

L'orifice central 34 présente successivement, en partant de la surface d'articulation convexe 30 en direction de la cavité principale 31 (et donc en direction de l'embase 2) :
- une portion proximale 340 cylindrique et non taraudée (par exemple lisse) et débouchant dans la surface d'articulation convexe 30 ; et
- une portion distale 341 munie d'un taraudage et débouchant dans la cavité principale 31.

La portion distale 341 est de diamètre réduit comparativement à la portion proximale 340, de sorte que l'orifice central 34 présente une portée annulaire 342 entre la fin de la portion proximale 340 et le début du taraudage de la portion distale 341.

L'implant glénoïdien 1 comprend en outre une vis de verrouillage 8 prévue pour traverser l'orifice central 34 de la glénosphère 3 afin de :
- guider la glénosphère 3 sur l'embase 2 pour faire coopérer la face interne périphérique 33 et la face périphérique 42 de la platine 4 pour un montage en cône morse ; et
- verrouiller la glénosphère 3 sur l'embase 2 dans une position verrouillée.

Cette vis de verrouillage 8 comprend une tête proximale 80 et une tige de verrouillage 81 centrée sur un axe longitudinal 89. En situation, la vis de verrouillage 8 est coaxiale à la vis d'ancrage principale 6, autrement dit l'axe longitudinal 89 et l'axe longitudinal 69 sont coaxiaux ou confondus.

La tête proximale 80 présente une face périphérique 82 externe cylindrique et non filetée (par exemple lisse), dont le diamètre externe est sensiblement équivalent (au jeu de montage près) au diamètre interne de la portion proximale 340 de l'orifice central 34 afin que cette tête proximale 80 puisse être guidée et montée ajustée à l'intérieur de cette portion proximale 340. La portion proximale 340 conformée pour recevoir intégralement la tête proximale 80 afin que celle-ci ne dépasse pas de la surface d'articulation convexe 30.

La tête proximale 80 présente également un trou borgne 83 conformée pour coopérer avec un outil de vissage, un tel trou borgne 83 pouvant par exemple être de section polygonale et notamment de section hexagonale pour coopérer avec une clé à six pans.

La tige de verrouillage 81 présente successivement, en partant de la tête proximale 80 :
- un segment proximal 83 cylindrique et non fileté (par exemple lisse) ;
- un segment intermédiaire 84 muni d'un filetage extérieur qui est complémentaire à la fois du taraudage de la portion intermédiaire 671 du trou interne 67 de la vis d'ancrage principale 6 et du taraudage de la portion distale 341 de l'orifice central 34 de la glénosphère 3 ; et
- un segment d'extrémité 85 cylindrique et non fileté (par exemple lisse) et qui se termine par une extrémité libre 86.

Le segment d'extrémité 85 présente une longueur LE qui est supérieure à une longueur cumulée LC de la portion d'entrée 670 et de la portion intermédiaire 671 du trou interne 67 de la vis d'ancrage principale 6, de sorte que ce segment d'extrémité 85 est prévu pour glisser à l'intérieur de la portion terminale 672 du trou interne 67 de la vis d'ancrage principale 6. En outre, le segment d'extrémité 85 présente un diamètre externe sensiblement équivalent (au jeu de montage près) au diamètre interne de la portion terminale 672 du trou interne 67 afin que ce segment d'extrémité 85 puisse être guidée et montée ajustée à l'intérieur de cette portion terminale 672.

Ainsi, en glissant à l'intérieur de la portion terminale 672 du trou interne 67, le segment d'extrémité 85 permet de centrer les portées coniques (ie. la face interne périphérique 33 et la face périphérique 42) entre elles, puis le segment intermédiaire 84 coopère par vissage avec la portion intermédiaire 671 du trou interne 67 de la vis d'ancrage principale 6 pour verrouiller le coincement mutuel des portées coniques 33, 42 jusqu'à ce que la tête proximale 80 de la vis de verrouillage 8 soit en butée sur la portée annulaire 342 prévue à l'intérieur de l'orifice central 34 de la glénosphère 3.

Il est à noter que la portion distale 341 de l'orifice central 34 de la glénosphère 3 présente une longueur inférieure à celle du segment intermédiaire 84 de la tige de verrouillage 81 de la vis de verrouillage 8, et également inférieure à celle du segment proximal 83 de la tige de verrouillage 81.

La suite de la description porte sur le procédé d'utilisation d'un implant glénoïdien 1 tel que décrit ci-dessus, le procédé ne faisant pas partie de l'invention.

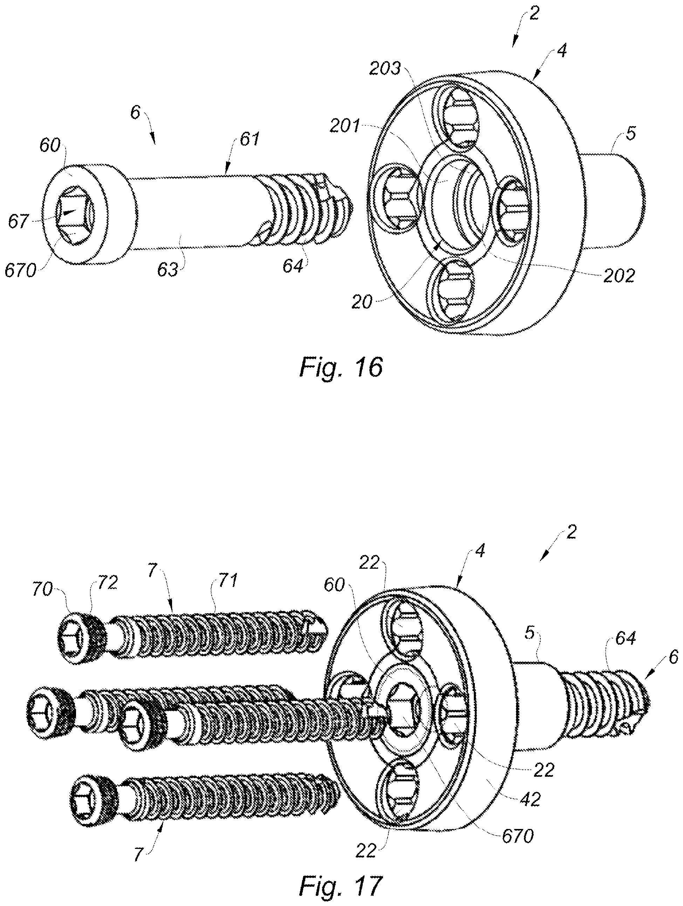

Dans une première étape illustrée sur la figure 16, la vis d'ancrage principale 6 est engagée dans l'orifice principal 20 de l'embase 2, jusqu'à ce que la tête principale 60 soit vissée dans la portion d'entrée 201 de l'orifice principal 20 jusqu'à venir en butée sur la portée annulaire 203 dans une position verrouillée visible sur les figures 21 à 23.

Dans une deuxième étape illustrée sur la figure 17, les vis d'ancrage principale 6 sont engagées dans les orifices secondaires 22 de l'embase 2, jusqu'à ce que les têtes secondaires 70 soient vissées dans les portions d'entrée 221 des orifices secondaires 22.

Dans une troisième étape illustrée sur la figure 21, la vis de verrouillage 8 est engagée dans l'orifice central 34 de la glénosphère 3 jusqu'à ce que le segment intermédiaire 84 entre dans la portion distale 341 de l'orifice central 34, et alors la vis de verrouillage 8 est vissée jusqu'à ce que le segment intermédiaire 84 dépasse de la portion distale 341 de sorte que le segment d'extrémité 85 de la tige de verrouillage 81 dépasse de la glénosphère 3 hors de la cavité principale 31.

De cette manière, la vis de verrouillage 8 est bloquée dans l'orifice central 34 de la glénosphère 3.

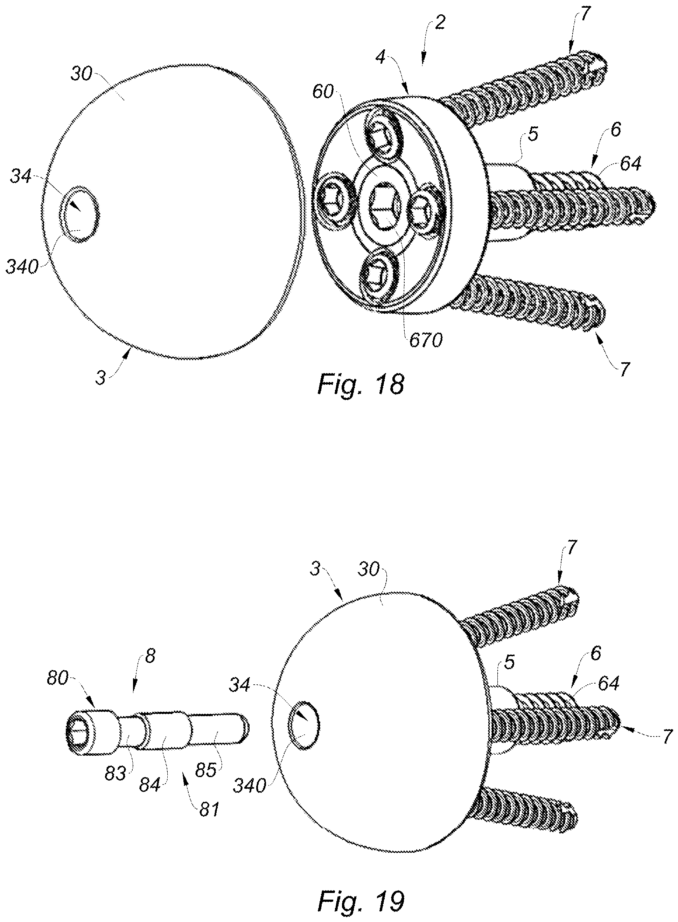

Cette troisième étape peut être effectuée après avoir positionné la glénosphère 3 sur l'embase 2, ainsi qu'illustré sur les figures 18 à 20.

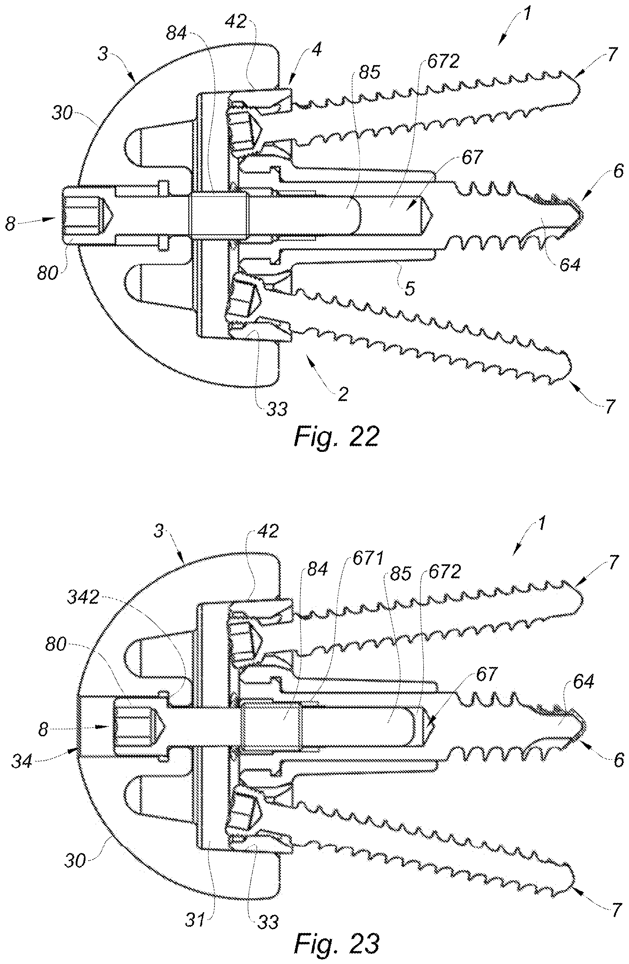

Dans une quatrième étape (qui peut précéder ou suivre la troisième étape) illustrée sur la figure 22, la glénosphère 3 est rapproché et monté sur l'embase 2 de sorte que :
- les portées coniques femelle et mâle (ie. la face interne périphérique 33 et la face périphérique 42) viennent au contact l'une de l'autre ; et
- le segment d'extrémité 85 de la tige de verrouillage 81 passe à travers la portion d'entrée 670 et la portion intermédiaire 671 du trou interne 67 de la vis d'ancrage principale 6 pour venir glisser à l'intérieur de la portion terminale 672 du trou interne 67, procurant ainsi un centrale pour les portées coniques femelle et mâle 33, 42.

A ce stade, la tête proximale 80 de la vis de verrouillage 8 est éloignée de la portée annulaire 342 prévue à l'intérieur de l'orifice central 34 de la glénosphère 3, ce qui autorise une impaction sur la glénosphère 3.

Ainsi, dans une cinquième étape, la glénosphère 3 est impactée pour assurer un coincement mutuel stable et fiable entre les portées coniques femelle et mâle 33, 42.

Enfin, dans une cinquième étape illustrée sur la figure 23, la tige de verrouillage 81 est enfoncée jusqu'à ce que le segment intermédiaire 84 entre dans la portion intermédiaire 671 du trou interne 67 de la vis d'ancrage principale 6, et alors la vis de verrouillage 8 est vissée pour que ce segment intermédiaire 84 coopère par vissage avec cette portion intermédiaire 671 du trou interne 67 de la vis d'ancrage principale 6 jusqu'à ce que la tête proximale 80 de la vis de verrouillage 8 soit en butée sur la portée annulaire 342 prévue à l'intérieur de l'orifice central 34 de la glénosphère 3. Pendant ce vissage, le segment d'extrémité 85 de la tige de verrouillage 81 continue de glisser à l'intérieur de la portion terminale 672 du trou interne 67.

Au final, la vis de verrouillage 8 procure le verrouillage du coincement mutuel des portées coniques femelle et mâle 33, 42, étant noté que la platine 4 s'étend au moins partiellement à l'intérieur de la cavité principale 31 de la glénosphère 3.

## Revendications

1. Implant glénoïdien (1) pour prothèse d'épaule inversée, comprenant :
- une embase (2) prévue pour être ancrée sur une glène d'une omoplate et présentant un orifice principal (20) traversant l'embase (2) ;
- une glénosphère (3) définissant une surface d'articulation convexe (30) en portion sphérique et présentant un orifice central (34) traversant la glénosphère (3), où ladite glénosphère (3) est montée sur ladite embase (2) par un coincement mutuel entre des portées coniques femelle et mâle (33, 42) prévues sur la glénosphère (3) et sur l'embase (2) ;
- une vis d'ancrage principale (6) traversant l'orifice principal (20) de ladite embase (2) et conformée pour l'ancrage de l'embase (2) sur la glène, où ladite vis d'ancrage principale (6) comprend une tête principale (60) et une tige d'ancrage (61) présentant un segment d'extrémité (64) muni d'un filetage extérieur (65) ;
- une vis de verrouillage (8), coaxiale à la vis d'ancrage principale (6), traversant l'orifice central (34) de la glénosphère (3) pour verrouiller la glénosphère (3) sur l'embase (2) dans une position verrouillée, où ladite vis de verrouillage (8) comprend une tête proximale (80) et une tige de verrouillage (81) présentant un segment intermédiaire (84) muni d'un filetage extérieur et prolongé par un segment d'extrémité (85) ;
dans lequel
- la vis d'ancrage principale (6) présente un trou interne (67) débouchant dans la tête principale (60) et présentant successivement, en partant de la tête principale (60) et en direction du segment d'extrémité (64) de la tige d'ancrage (61), une portion d'entrée (670) conformée pour coopérer avec un outil de vissage, une portion intermédiaire (671) munie d'un taraudage et une portion terminale (672) ;
- la tige de verrouillage (81) a son segment d'extrémité (85) qui présente une longueur (LE) supérieure à une longueur cumulée (LC) de la portion d'entrée (670) et de la portion intermédiaire (671) du trou interne (67) de la vis d'ancrage principale (6), de sorte que ledit segment d'extrémité (85) de la tige de verrouillage (81) est prévu pour glisser à l'intérieur de la portion terminale (672) du trou interne (67) de la vis d'ancrage principale (6) pour centrer les portées coniques femelle et mâle (33, 42) entre elles, et que le segment intermédiaire (84) de la tige de verrouillage (81) est prévu pour ensuite coopérer par vissage avec la portion intermédiaire (671) du trou interne (67) de la vis d'ancrage principale (6) pour verrouiller le coincement mutuel des portées coniques femelle et mâle (33, 42), la tête proximale (80) de la vis de verrouillage (8) étant en butée sur une portée annulaire (342) prévue à l'intérieur de l'orifice central (34) de la glénosphère (3) et dans lequel l'orifice central (34) de la glénosphère (3) présente successivement, en partant de la surface d'articulation convexe (30) en direction de l'embase (2), une portion proximale (340) conformée pour recevoir intégralement la tête proximale (80) de la vis de verrouillage (8), et une portion distale (341) munie d'un taraudage complémentaire du filetage extérieur du segment intermédiaire (84) de la tige de verrouillage (81) de la vis de verrouillage (8), où la portée annulaire (342) de l'orifice central (34) de la glénosphère (3) est prévue à l'interface entre ladite portion proximale (340) et ladite portion distale (341).

2. Implant glénoïdien (1) selon la revendication 1, dans lequel la portion distale (341) de l'orifice central (34) de la glénosphère (3) présente une longueur inférieure à celle du segment intermédiaire (84) de la tige de verrouillage (81) de la vis de verrouillage (8).

3. Implant glénoïdien (1) selon l'une quelconque des revendications précédentes, dans lequel la tige de verrouillage (81) de la vis de verrouillage (8) présente un segment proximal (83) s'étendant entre la tête proximale (80) et le segment intermédiaire (84).

4. Implant glénoïdien (1) selon la revendication 3, dans lequel la portion distale (341) de l'orifice central (34) de la glénosphère (3) présente une longueur inférieure à celle du segment proximale (83) de la tige de verrouillage (81) de la vis de verrouillage (8).

5. Implant glénoïdien (1) selon l'une quelconque des revendications précédentes, dans lequel la vis d'ancrage principale (6) et la vis de verrouillage (8) sont coaxiales aux portées coniques femelle et mâle (33, 42).

6. Implant glénoïdien (1) selon l'une quelconque des revendications précédentes, dans lequel, en position verrouillée, la tête principale (60) de la vis d'ancrage principale (6) vient en butée sur une portée annulaire (203) prévue à l'intérieur de l'orifice principal (20) de l'embase (2).

7. Implant glénoïdien (1) selon l'une quelconque des revendications précédentes, dans lequel la tête principale (60) de la vis d'ancrage principale (6) présente un filetage extérieur (62), et l'orifice principal (20) de l'embase (2) présente une portion d'entrée (201) débouchant sur une face externe (40) de l'embase (2) faisant face à la glénosphère (3), où ladite portion d'entrée (201) est munie d'un taraudage complémentaire dudit filetage extérieur (62) de ladite tête principale (60), de sorte que ladite tête principale (60) est vissée dans ladite portion d'entrée (201).

8. Implant glénoïdien (1) selon la revendication précédente, dans lequel le filetage extérieur (62) de la tête principale (60) de la vis d'ancrage principale (6) présente un pas de filetage inférieur à celui du filetage extérieur (65) du segment d'extrémité (64) de la tige d'ancrage (61).

9. Implant glénoïdien (1) selon l'une quelconque des revendications précédentes, dans lequel l'embase (2) présente un plot central (5) saillant d'une face interne (41) opposée à la glénosphère (3), où ledit plot central (5) est creux et est traversé par l'orifice principal (20) et la vis d'ancrage principale (6) dont le segment d'extrémité (64) de la tige d'ancrage (61) dépasse dudit plot central (5).

10. Implant glénoïdien (1) selon les revendications 7 et 9, dans lequel l'orifice principal (20) de l'embase (2) présente une portion de sortie (202) qui prolonge la portion d'entrée (201) et qui traverse le plot central (5), et la tige d'ancrage (61) présente un segment intermédiaire (63) conformé pour être monté glissant et ajusté à l'intérieur de la portion de sortie (202) de l'orifice principal (20) de l'embase (2).

11. Implant glénoïdien (1) selon l'une quelconque des revendications précédentes, comprenant en outre plusieurs vis d'ancrage secondaires (7) conformées pour l'ancrage de l'embase (2) sur la glène, où lesdites vis d'ancrage secondaires (7) traversent l'embase (2) et sont disposées en périphérie autour de la vis d'ancrage principale (6).

12. Implant glénoïdien (1) selon l'une quelconque des revendications précédentes, dans lequel la tête principale (60) de la vis d'ancrage principale (6) ne dépasse pas d'une face externe (40) faisant face à la glénosphère (3).

13. Prothèse d'épaule inversée comprenant un implant glénoïdien (1) conforme à l'une quelconque des revendications précédentes, et un implant huméral prévu pour être ancré sur un humérus et comprenant une calotte hémisphérique définissant une surface d'articulation concave conformée pour une articulation avec la surface d'articulation convexe (30) de la glénosphère (3) de l'implant glénoïdien (1).

## Patentansprüche

1. Glenoidimplantat (1) für eine umgekehrte Schulterprothese, umfassend:
- eine Basis (2), die dazu vorgesehen ist, an einer Glenoidpfanne eines Schulterblatts verankert zu werden, und eine Hauptöffnung (20) aufweist, die durch die Basis (2) verläuft;
- eine Glenosphäre (3), die eine konvexe Gelenkfläche (30) aus einem kugelförmigen Abschnitt definiert und eine zentrale Öffnung (34) aufweist, die durch die Glenosphäre (3) verläuft, wobei die Glenosphäre (3) an der Basis (2) montiert wird durch gegenseitiges Einklemmen zwischen einem weiblichen und einem männlichen konischen Lagersitz (33, 42), die an der Glenosphäre (3) und an der Basis (2) vorgesehen sind;
- eine Hauptverankerungsschraube (6), die durch die Hauptöffnung (20) der Basis (2) verläuft und zur Verankerung der Basis (2) in der Glenoidpfanne ausgestaltet ist, wobei die Hauptverankerungsschraube (6) einen Hauptkopf (60) und eine Verankerungsstange (61) umfasst, die ein mit einem Außengewinde (65) versehenes Endsegment (64) aufweist; und
- eine Verriegelungsschraube (8), die koaxial zur Hauptverankerungsschraube (6) ist, durch die zentrale Öffnung (34) der Glenosphäre (3) verläuft, um die Glenosphäre (3) in einer verriegelten Position an der Basis (2) zu verriegeln, wobei die Verriegelungsschraube (8) einen proximalen Kopf (80) und eine Verriegelungsstange (81) umfasst, die ein mit einem Außengewinde versehendes Zwischensegment (84) aufweist und um ein Endsegment (85) verlängert ist;
wobei:
- die Hauptverankerungsschraube (6) ein Innenloch (67) aufweist, das sich in den Hauptkopf (60) öffnet und nacheinander, ausgehend vom Hauptkopf (60) und in Richtung des Endsegments (64) der Verankerungsstange (61), einen Einlassabschnitt (670), der so ausgestaltet ist, dass er mit einem Schraubwerkzeug zusammenwirkt, und einen Zwischenabschnitt (671) aufweist, der mit einem Innengewinde und einem Endabschnitt (672) versehen ist;
- die Verriegelungsstange (81) sein Endsegment (85) aufweist, das eine Länge (LE) aufweist, die größer ist als die Gesamtlänge (LC) des Einlassabschnitts (670) und des Zwischenabschnitts (671) des Innenlochs (67) der Hauptverankerungsschraube (6), so dass der Endabschnitt (85) der Verriegelungsstange (81) dazu vorgesehen ist, im Inneren des Endabschnitts (672) des Innenlochs (67) der Hauptverankerungsschraube (6) zu gleiten, um den weiblichen und den männlichen konischen Lagersitz zueinander zu zentrieren, und dass das Zwischensegment (84) der Verriegelungsstange (81) dazu vorgesehen ist, durch Schrauben mit dem Zwischenabschnitt (671) des Innenlochs (67) der Hauptverankerungsschraube (6) anschließend zusammenzuwirken, um das gegenseitige Einklemmen des weiblichen und des männlichen konischen Lagersitzes (33, 42) zu verriegeln, wobei der proximale Kopf (80) der Verriegelungsschraube (8) an einem ringförmigen Lagersitz (342) anliegt, der im Inneren der zentralen Öffnung (34) der Glenosphäre (3) vorgesehen ist,
und wobei die zentrale Öffnung (34) der Glenosphäre (3) nacheinander, ausgehend von der konvexen Gelenkfläche (30) in Richtung der Basis (2), einen proximalen Abschnitt (340), der so ausgestaltet ist, dass er den proximalen Kopf (80) der Verriegelungsschraube (8) aufnimmt, und einen distalen Abschnitt (341) aufweist, der mit einem zum Außengewinde des Zwischensegments (84) der Verriegelungsstange (81) der Verriegelungsschraube (8) komplementären Innengewinde versehen ist, wobei der ringförmige Lagersitz (342) der zentralen Öffnung (34) der Glenosphäre (3) an einer Schnittstelle zwischen dem proximalen Abschnitt (340) und dem distalen Abschnitt (341) vorgesehen ist.

2. Glenoidimplantat (1) nach Anspruch 1, wobei der distale Abschnitt (341) der zentralen Öffnung (34) der Glenosphäre (3) eine Länge aufweist, die kleiner ist als die des Zwischensegments (84) der Verriegelungsstange (81) der Verriegelungsschraube (8).

3. Glenoidimplantat (1) nach einem der vorhergehenden Ansprüche, wobei die Verriegelungsstange (81) der Verriegelungsschraube (8) ein proximales Segment (83) aufweist, das sich zwischen dem proximalen Kopf (80) und dem Zwischensegment (84) erstreckt.

4. Glenoidimplantat (1) nach Anspruch 3, wobei der distale Abschnitt (341) der zentralen Öffnung (34) der Glenosphäre (3) eine Länge aufweist, die kleiner ist als die des proximalen Segments (83) der Verriegelungsstange (81) der Verriegelungsschraube (8).

5. Glenoidimplantat (1) nach einem der vorhergehenden Ansprüche, wobei die Hauptverankerungsschraube (6) und die Verriegelungsschraube (8) koaxial zu dem weiblichen und dem männlichen konischen Lagersitz (33, 42) sind.

6. Glenoidimplantat (1) nach einem der vorhergehenden Ansprüche, wobei der Hauptkopf (60) der Hauptverankerungsschraube (6) in der verriegelten Position an einem ringförmigen Lagersitz (203) zum Anliegen kommt, der im Inneren der Hauptöffnung (20) der Basis (2) vorgesehen ist.

7. Glenoidimplantat (1) nach einem der vorhergehenden Ansprüche, wobei der Hauptkopf (60) der Hauptverankerungsschraube (6) ein Außengewinde (62) aufweist und die Hauptöffnung (20) der Basis (2) einen Einlassabschnitt (201) aufweist, der sich zu einer Außenfläche (40) der Basis (2) gegenüber der Glenosphäre (3) öffnet, wobei der Einlassabschnitt (201) mit einem zum Außengewinde (62) des Hauptkopfes (60) komplementären Innengewinde versehen ist, so dass der Hauptkopf (60) in den Einlassabschnitt (201) eingeschraubt wird.

8. Glenoidimplantat (1) nach dem vorhergehenden Anspruch, wobei das Außengewinde (62) des Hauptkopfs (60) der Hauptverankerungsschraube (6) eine Gewindesteigung aufweist, die kleiner ist als die des Außengewindes (65) des Endsegments (64) der Verankerungsstange (61).

9. Glenoidimplantat (1) nach einem der vorhergehenden Ansprüche, wobei die Basis (2) einen zentralen Zapfen (5) aufweist, der von einer der Glenosphäre (3) gegenüberliegenden Innenfläche (41) vorsteht, wobei der zentrale Zapfen (5) hohl ist und von der Hauptöffnung (20) und der Hauptverankerungsschraube (6) durchquert wird, deren Endsegment (64) der Verankerungsstange (61) über den zentralen Zapfen (5) hinausragt.

10. Glenoidimplantat (1) nach den Ansprüchen 7 und 9, wobei die Hauptöffnung (20) der Basis (2) einen Auslassabschnitt (202) aufweist, der den Einlassabschnitt (201) verlängert und der durch den zentralen Zapfen (5) verläuft, und die Verankerungsstange (61) ein Zwischensegment (63) aufweist, das so ausgestaltet ist, dass es im Inneren des Auslassabschnitts (202) der Hauptöffnung (20) der Basis (2) gleitend montiert und justiert ist.

11. Glenoidimplantat (1) nach einem der vorhergehenden Ansprüche, ferner umfassend mehrere sekundäre Verankerungsschrauben (7), die zur Verankerung der Basis (2) in der Glenoidpfanne ausgestaltet sind, wobei die sekundären Verankerungsschrauben (7) durch die Basis (2) verlaufen und an einem Umfang um die Hauptverankerungsschraube (6) herum angeordnet sind.

12. Glenoidimplantat (1) nach einem der vorhergehenden Ansprüche, wobei der Hauptkopf (60) der Hauptverankerungsschraube (6) nicht über eine der Glenosphäre (3) gegenüberliegende Außenfläche (40) hinausragt.

13. Umgekehrte Schulterprothese, umfassend ein Glenoidimplantat (1), das nach einem der vorhergehenden Ansprüche ausgestaltet ist, und ein Humerusimplantat, das dazu vorgesehen ist, an einem Oberarmknochen verankert zu werden, und eine halbkugelförmige Kalotte umfasst, die eine konkave Gelenkfläche definiert, die für ein Gelenk mit der konvexen Gelenkfläche (30) der Glenosphäre (3) des Glenoidimplantats (1) ausgestaltet ist.

## Claims

1. A glenoid implant (1) for an inverted shoulder prosthesis, comprising:
- a base (2) intended to be anchored on a glenoid cavity of a scapula and having a main orifice (20) crossing the base (2);
- a glenosphere (3) defining a convex articulation surface (30) formed in a spherical portion and having a central orifice (34) crossing the glenosphere (3), wherein said glenosphere (3) is mounted on said base (2) by mutual wedging between female and male conical bearing surfaces (33, 42) provided on the glenosphere (3) and on the base (2);
- a main anchoring screw (6) crossing the main orifice (20) of said base (2) and shaped for anchorage of the base (2) on the glenoid cavity, wherein said main anchoring screw (6) comprises a main head (60) and an anchor rod (61) having an end segment (64) provided with an external tapping (65);
- a locking screw (8), coaxial with the main anchoring screw (6), crossing the central orifice (34) of the glenosphere (3) to lock the glenosphere (3) on the base (2) in a locked position, wherein said locking screw (8) comprises a proximal head (80) and a locking rod (81) having an intermediate segment (84) provided with an external tapping and extended by an end segment (85);
wherein:
- the main anchoring screw (6) has an inner hole (67) opening into the main head (60) and having successively, starting from the main head (60) and in the direction of the end segment (64) of the anchor rod (61), an inlet portion (670) shaped so as to cooperate with a screwing tool, an intermediate portion (671) provided with a threading and a tip portion (672);
- the locking rod (81) has its end segment (85) which has a length (LE) longer than a cumulated length (LC) of the inlet portion (670) and of the intermediate portion (671) of the inner hole (67) of the main anchoring screw (6), so that said end segment (85) of the locking rod (81) is intended to slide inside the tip portion (672) of the inner hole (67) of the main anchoring screw (6) to center the female and male conical bearing surfaces (33, 42) relative to one another, and the intermediate segment (84) of the locking rod (81) is intended to cooperate afterwards by screwing with the intermediate portion (671) of the inner hole (67) of the main anchoring screw (6) to lock the mutual wedging of the female and male conical bearing surfaces (33, 42), the proximal head (80) of the locking screw (8) abutting on an annular bearing surface (342) provided inside the central orifice (34) of the glenosphere (3),
and wherein the central orifice (34) of the glenosphere (3) has successively, starting from the convex articulation surface (30) in the direction of the base (2), a proximal portion (340) shaped so as to completely receive the proximal head (80) of the locking screw (8), and a distal portion (341) provided with a threading complementary to the external tapping of the intermediate segment (84) of the locking rod (81) of the locking screw (8), wherein the annular bearing surface (342) of the central orifice (34) of the glenosphere (3) is provided at the interface between said proximal portion (340) and said distal portion (341).

2. The glenoid implant (1) according to claim 1, wherein the distal portion (341) of the central orifice (34) of the glenosphere (3) has a length smaller than that of the intermediate segment (84) of the locking rod (81) of the locking screw (8).

3. The glenoid implant (1) according to any one of the preceding claims, wherein the locking rod (81) of the locking screw (8) has a proximal segment (83) extending between the proximal head (80) and the intermediate segment (84).

4. The glenoid implant (1) according to claim 3, wherein the distal portion (341) of the central orifice (34) of the glenosphere (3) has a length smaller than that of the proximal segment (83) of the locking rod (81) of the locking screw (8).

5. The glenoid implant (1) according to any one of the preceding claims, wherein the main anchoring screw (6) and the locking screw (8) are coaxial with the female and male conical bearing surfaces (33, 42).

6. The glenoid implant (1) according to any one of the preceding claims, wherein, in the locked position, the main head (60) of the main anchoring screw (6) abuts on an annular bearing surface (203) provided inside the main orifice (20) of the base (2).

7. The glenoid implant (1) according to any one of the preceding claims, wherein the main head (60) of the main anchoring screw (6) has an external tapping (62), and the main orifice (20) of the base (2) has an inlet portion (201) opening onto an outer face (40) of the base (2) opposite the glenosphere (3), wherein said inlet portion (201) is provided with a threading complementary to said external tapping (62) of said main head (60), so that said main head (60) is screwed into said inlet portion (201).

8. The glenoid implant (1) according to the preceding claim, wherein the external tapping (62) of the main head (60) of the main anchoring screw (6) has a tapping pitch smaller than that of the external tapping (65) of the end segment (64) of the anchor rod (61).

9. The glenoid implant (1) according to any one of the preceding claims, wherein the base (2) has a central stud (5) projecting from an inner face (41) opposite to the glenosphere (3), wherein said central stud (5) is hollow and is crossed by the main orifice (20) and the main anchoring screw (6) whose end segment (64) of the anchor rod (61) projects beyond said central stud (5).

10. The glenoid implant (1) according to claims 7 and 9, wherein the main orifice (20) of the base (2) has an outlet portion (202) which extends the inlet portion (201) and which crosses the central stud (5), and the anchor rod (61) has an intermediate segment (63) shaped so as to be slidably and tightly mounted inside the outlet portion (202) of the main orifice (20) of the base (2).

11. The glenoid implant (1) according to any one of the preceding claims, further comprising several secondary anchoring screws (7) shaped for anchorage of the base (2) on the glenoid cavity, wherein said secondary anchoring screws (7) cross the base (2) and are disposed at the periphery around the main anchoring screw (6).

12. The glenoid implant (1) according to any one of the preceding claims, wherein the main head (60) of the main anchoring screw (6) does not project beyond an outer face (40) opposite the glenosphere (3).

13. An inverted shoulder prosthesis comprising a glenoid implant (1) in accordance with any one of the preceding claims, and a humeral implant intended to be anchored on a humerus and comprising a hemispherical cap defining a concave articulation surface shaped for articulation with the convex articulation surface (30) of the glenosphere (3) of the glenoid implant (1).
